# EUROPEAN PATENT APPLICATION

(11) **EP 1 535 629 A1**
(43) Date of publication of application: **01.06.2005**
(21) Application number: 03764186.7
(22) Date of filing: 14.07.2003
(51) Int. Cl.: A61K 45/00, A61K 31/454, A61K 31/53, A61K 31/381, A61P 25/00, A61P 43/00

(54) **DRUGS FOR IMPROVING THE PROGNOSIS OF BRAIN INJURY AND A METHOD OF SCREENING THE SAME**

(30) Priority: 12.07.2002 JP 2002204725; 16.01.2003 JP 2003008230
(71) Applicant: Japan Science and Technology Agency, Kawaguchi-shi, Saitama 332-0012 (JP); OSAKA BIOSCIENCE INSTITUTE, Suita-shi, Osaka-Fu 565-0874 (JP); TAIHO PHARMACEUTICAL COMPANY LIMITED, Chiyoda-ku, Tokyo 101-8444 (JP); SHIONOGI & CO., LTD., Osaka-shi, Osaka 541-0045 (JP)
(72) Inventor: URADE, Y., 503, Granshitio-Shimogamo-Shikisaikan, Kyoto-shi, Kyoto 606-0804 (JP); EGUCHI, Naomi, Suita-shi, Osaka 565-0821 (JP); ARITAKE, Kosuke, Kawanishi-shi, Hyogo 666-0143 (JP); SATO, Yo, Mino-shi, Osaka 562-0031 (JP); KADOYAMA, Keiichi, Suita-shi, Osaka 565-0821 (JP); TANIIKE, Masako, Itami-shi, Hyogo 664-0014 (JP)
(74) Representative: Hayes, Adrian Chetwynd
(86) International application number: PCT/JP2003/008904
(87) International publication number: WO 2004/006956

(57) **Abstract**

There are provided a compound for treatment or prevention of brain injury caused by diseases such as cerebrovascular disorder, brain degenerative disease and demyelinating disease and a method for screening the same.

Brain injury in which prostaglandin D₂ is participated is treated or prevented by inhibition of hematopoietic prostaglandin D synthase induced in microglia cell or macrophage of brain injury area by diseases such as cerebrovascular disorder, brain degenerative disease or demyelinating disease or by inhibition of activation of prostaglandin D receptor expressed in astroglia cell around the injured area. There is also provided a method of testing those pharmaceutical substances using a transgenic mouse in which human hematopoietic prostaglandin D synthase is expressed in large amounts.

## Description

### TECHNICAL FIELD

This invention relates to a compound for treating or preventing brain injury and to a method of screening the same. More particularly, this invention relates to a compound which treats or prevents brain injury in which prostaglandin D₂ is participated by inhibition of hematopoietic prostaglandin D synthase (hereinafter, may be referred to as "H-PGDS") induced in microglia cell or macrophage of brain injury site by diseases such as cerebrovascular accident, neurodegenerative disease or demyelinating disease or by inhibition of activation of prostaglandin D receptor (hereinafter, may be referred to as "DP receptor") expressed in astroglial cell around the injured site and also relates to a method of testing those pharmaceutical substances using a human hematopoietic prostaglandin D synthase over-expressing transgenic mouse.

### BACKGROUND ART

As a result of development of medical technology, people having a narrow escape from death even if the head suffered from a serious injury are increasing. There are many people who suffer from head injury by traffic accidents, work-related accidents, sports, etc. and, in fact, one half of the people who are dead by traffic accidents are caused by head injury. They are caused by brain edema or brain contusion generated immediately beneath the cranium when it is pressed by external force. Cause for brain edema is breakage of blood-brain barrier existing in cerebral blood vessel and is edema of brain caused by leakage of plasma components outside the blood vessel. In recent years, hypothermia has been receiving public attention as a treating method therefor and that is a treating method where exacerbation of brain edema and increase in pressure in the cranium are suppressed by suppression of brain metabolism whereby the secondary brain injury is intended to prevent. Although that is an important treatment policy, there are some cases where problems by lowering of cardiopulmonary function and immunological function due to low body temperature occur (*N*. *Engl. J. Med.,* 2001; 344:556-563).

### DISCLOSURE OF THE INVENTION

An obj ect of the present invention is to provide a compound which is able to suppress the tissue injury by delay of local inflammation of brain and to improve the prognosis.

Another object of the present invention is to provide a method of screening such a compound.

In order to achieve the above-mentioned objects, the present inventors have carried out intensive studies and found the followings and, on the basis thereof, the present invention has been accomplished.
1) When hereditary or traumatic brain injuries occurs, expression of H-PGDS and DP receptor increases.
2) Expression of H-PGDS is induced in activated microglia cell or accumulated macrophage of brain injury while DP receptor is induced in astroglia cell around the injured area.
3) In the injured area, accumulation of macrophage and activation of astroglia cell are significant.
4) When an inhibitor for H-PGDS or an antagonist for DP receptor is administered, expression of DP receptor decreases and activation of astroglia cell is suppressed.
5) In H-PGDS over-expressing transgenic mouse, brain injury is exacerbated as compared with a mouse of a wild type.
6) In an H-PGDS knockout mouse and DP receptor knockout mouse, bleeding at the injured site and activation of astroglia cell are slight as compared with a mouse of a wild type.

Thus, the gist of the present invention is a pharmaceutical composition used for treatment or prevention of brain injury containing a suppressor for hematopoietic prostaglandin D synthase (H-PGDS) as an active ingredient.

The term "brain injury" includes not only traumatic one caused by traffic accident or the like but also that caused by cerebrovascular disorder such as cerebral infarction and cerebral bleeding, neuronal degenerative disease including Alzheimer's disease, multiple sclerosis, etc. and demyelinating disease and the like. The term "treatment or prevention of brain injury" includes treatment or prevention of brain contusion, brain edema, cerebral infarction, cerebral bleeding, ischemic brain diseases, Alzheimer's disease, multiple sclerosis and demyelinating disease.

Another gist of the present invention is a pharmaceutical composition used for treatment or prevention of brain injury containing an antagonist for prostaglandin D receptor as an effective ingredient.

Still another gist of the present invention is a method for treatment of brain injury including administration of a hematopoietic prostaglandin D synthase (H-PGDS) inhibitor of an effective dose.

Still another object of the present invention is the use of a hematopoietic prostaglandin D synthase (H-PGDS) inhibitor for the manufacture of a drug for treatment of brain injury.

Still another object of the present invention is a method for treatment of brain injury including administration of a prostaglandin D receptor antagonist of an effective dose.

Still another object of the present invention is the use of a prostaglandin D receptor antagonist for the manufacture of a drug for treatment of brain injury.

Still another object of the present invention is a pharmaceutical composition to be used for treatment or prevention of brain injury containing a hematopoietic prostaglandin D synthase (H-PGDS) inhibitor and a prostaglandin D receptor antagonist as effective ingredients.

Still another object of the present invention is a method of screening of a compound used for treatment or prevention of brain injury including that
1) trauma is applied to brain of a human H-PGDS over-expressing transgenic mouse,
2) a candidate compound is administered to the transgenic mouse before or after applying the trauma and
3) a state of the trauma in the mouse is compared with a state of a transgenic mouse to which no candidate compound is administered.

### BRIEF DESCRIPTION OF THE DRAWINGS

Fig. 1 shows the changes in expression level of H-PGDS and DP receptor mRNAs in the cerebrum and the cerebellum of Twitcher mice.
Fig. 2 shows H-PGDS locally existing in macrophage and microglia cells of Twitcher mice by an immunohistochemistry.
Fig. 3 shows DP receptors expressed in activated astroglia cells.
Fig. 4 shows the changes in expression level of H-PGDS and DP receptor mRNAs in mice suffering from experimental autoimmune encephalomyelitis.
Fig. 5 shows the changes in H-PGDS expression level in mice suffering from experimental autoimmune encephalomyelitis.
Fig. 6 shows the time course of H-PGDS mRNA expression in traumatic brain injury models.
Fig. 7 shows the time course of DP receptor mRNA expression in traumatic brain injury models.
Fig. 8 shows the time course of tissue edema in traumatic brain injury models.
Fig. 9 shows the time course of H-PGDS expression in traumatic brain injury models.
Fig. 10 shows the time course of astroglia-activation (GFAP activities) in traumatic brain injury models.
Fig. 11 shows expression of DP receptor in activated astroglia cells around the injured brain.
Fig. 12 shows comparison of brain injury in a wild-type mouse after 4 days from traumatic brain injury with that in a human H-PGDS over-expressing transgenic mouse.
Fig. 13 shows suppression of DP receptor expression and activation of astroglia cells in HQL-79-treated Twitcher mouse.
Fig. 14 shows an inhibitory effect of HQL-79 on DP receptor expression after traumatic brain injury.
Fig. 15 shows a recovery-promoting effect of HQL-79 on traumatic brain injury.
Fig. 16 shows a recovery-promoting effect of DP receptor antagonist on traumatic brain injury.
Fig. 17 shows comparison of brain injury in a wild-type mouse after 4 days from traumatic brain injury with that in a H-PGDS knockout.
Fig. 18 shows the structure of targeting vector used for the preparation of a transgenic mouse.
Fig. 19 shows the structure of mouse H-PGDS gene (upper column), the structure of mutant sequence in a targeting vector (middle column) and the structure of a mouse genome DNA after homologous recombination (lower column).
Fig. 20 shows the time course of brain edema in a traumatic brain injury model. The brain edema was evaluated by Evans Blue dye leakage into the tissues.
Fig. 21 shows an inhibitory effect of HQL-79 on tissue injury (leakage of dye) after a traumatic brain injury (Administration of HQL-79 was started before one hour from injury was applied).
Fig. 22 shows an inhibitory effect of HQL-79 on tissue injury after a traumatic brain injury (Administration of HQL-79 was started after 24 hours from injury was applied).
Fig. 23 shows an inhibitory effect of HQL-79 on tissue injury (leakage of dye) after a traumatic brain injury (Administration of HQL-79 was started after 24 hours from injury was applied).
Fig. 24 an inhibitory effect of pinagladin on leakage of Evans Blue dye as a result of a traumatic brain injury.
Fig. 25 shows an inhibitory effect on progress of a traumatic brain injury by pinagladin.
Fig. 26 shows a comparison of brain injury in a wild-type mouse after two days from a traumatic injury with that in an HPGDS KO or DPR KO mouse using leakage of dye into the injured site.
Fig. 27 shows inhibitory effects of BWA 868 and ramatroban on tissue injury (dye leakage) after a traumatic brain injury.

### DETAILED DESCRIPTION OF THE INVENTION

Examples of an H-PGDS inhibitor include 4-benzhydryloxy-1-{3-(1H-tetrazol-5-yl)-propyl}piperidine (HQL-79), 1-amino-4-{4-[4-chloro-6-(2-sulfo-phenylamino)-[1,3,5]-triazine-2-yl methyl]-3-sulfo-phenylamino}-9,10-dioxo-9,10-dihydro-anthracene-2-sulfonic acid (Cibacron Blue), 1-amino-4-(4-sulfamoylanilino)-anthraquinone-2-sulfonic acid (PGD-042) or pharmaceutically acceptable salt thereof or hydrate thereof and 2-(2'-benzothiazolyl)-5-styryl-3-(4'-phthalhydrazidyl)tetrazolium chloride (PGD-016) or a hydrate thereof.

In the present specification, examples of the "pharmaceutically acceptable salt" in the case of salt with base include alkaline metal salt such as sodium salt and potassium salt; alkaline earth metal salt such as calcium salt and magnesium salt; ammonium salt; aliphatic amine salt such as trimethylamine salt, triethylamine salt, dicyclohexylamine salt, ethanolamine salt, diethanolamine salt, triethanolamine salt and procaine salt; aralkylamine salt such as N,N-dibenzylethylenediamine salt; heterocyclic aromatic amine salt such as pyridine salt, picoline salt, quinoline salt and isoquinoline salt; quaternary ammonium salt such as tetramethylammonium salt, tetraethylammonium salt, benzyltrimethylammonium salt, benzyltriethylammonium salt, benzyltributylammonium salt, methyltrioctylammonium salt and tetrabutylammonium salt; and basic amino acid salt such as arginine salt and lysine salt. Examples in the case of salt with acid include inorganic acid salt such as hydrochloride, sulfate, nitrate, phosphate, carbonate, hydrogen carbonate and perchlorate; organic acid salt such as acetate, propionate, lactate, maleate, fumarate, tartrate, malate, citrate and ascorbate; sulfonate such as isothionate, benzenesulfonate and p-toluenesulfonate; and acidic amino acid salt such as aspartate and glutamate. Such a salt can be prepared by conventional methods. When a hydrate is formed, coordination with any number of water molecule(s) is acceptable.

Another embodiment of the present invention is a pharmaceutical composition comprising an antagonist for a prostaglandin D receptor as an active ingredient to be used for treatment or prevention of brain injury.

Examples of the antagonist for a prostaglandin D receptor are (±)-3-benzyl-5-(6-carboxyhexyl)-1-(2-cyclohexyl-2-hydroxyethylamino)-hydantoin (BW A868C), (+)-(3R)-3-(4-fluorobenzenesulfonamide)-1,2,3,4-tetrahydrocarbazol-9-propionic acid (ramatroban), (Z)-7-[(1R,2R,3S,5S)-2-(5-hydroxybenzo[b]thiophene-3-ylcarbonylamino)-10-norpinan-3-yl]hepta-5-enoic acid, (Z)-7-[(1R,2R,3S,5S)-2-(benzo[b]-thiophene-3-ylcarbonylamino)-10-norpinan-3-yl]hepta-5-enoic acid (pinagladin) and pharmaceutically acceptable salt thereof and hydrate thereof.

Further examples of the antagonist for a prostaglandin D receptor are a compound represented by the formula (I): 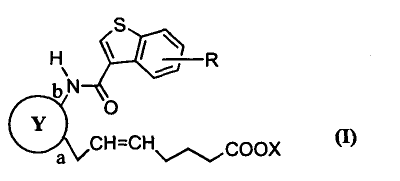 wherein 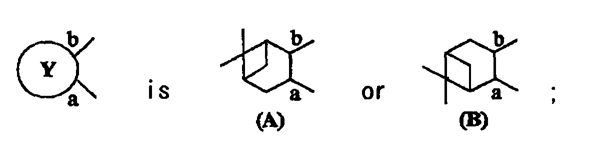 R is hydrogen, alkyl, alkoxy, halogen, hydroxyl, acyloxy or optionally substituted arylsulfonyloxy; X is hydrogen or alkyl; and a double bond of an α-chain is in an E-configuration or a Z-configuration) or a pharmaceutically acceptable salt or a hydrate thereof.

In a preferred embodiment, the antagonist for a prostaglandin D receptor is a compound represented by the formula (IA): 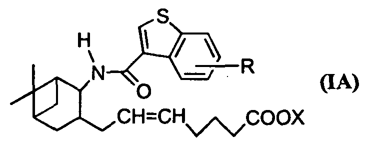 wherein R and X have the same meanings as defined above and a double bond of an α-chain is in an E-configuration or a Z-configuration, or a pharmaceutically acceptable salt or a hydrate thereof.

More preferably, the antagonist for a prostaglandin D receptor is a compound represented by the formula (IA-a): 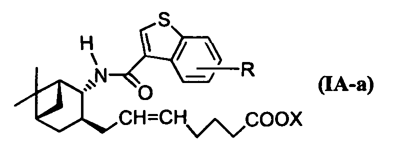 wherein R and X have the same meanings as defined above and a double bond of an α-chain is in an E-configuration or a Z-configuration, or a pharmaceutically acceptable salt or a hydrate thereof.

The compound represented by the formula (I) has been known and can be produced according to the following process: 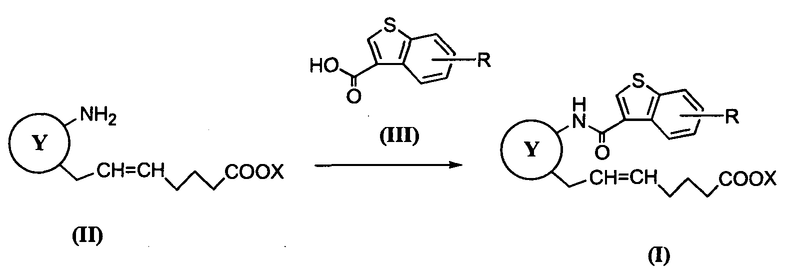 wherein Y ring, X and R have the same meanings as defined above and a double bond of an α-chain is in an E-configuration or a Z-configuration.

As shown in the above reaction formula, the compound represented by the formula (I) is able to be produced by the reaction of an amino compound represented by the formula (II) with a carboxylic acid or a reactive derivative thereof represented by the formula (III).

In the starting compound (II) in the present reaction method, a compound in which 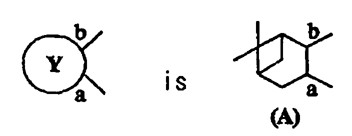 is described in the specification of the Japanese Patent Publication No. 23,170 (1994) B while a compound in which 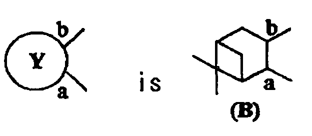 is described in the specifications of the Japanese Patent Laid-Open Nos. 49 (1986) A and 180,862 (1990) A.

The carboxylic acids represented by the formula (III) include 4-bromobenzo[b]thiophene-3-carboxylic acid, 5-bromobenzo[b]thiophene-3-carboxylic acid, 6-bromobenzo[b]thiophene-3-carboxylic acid, 7-bromobenzo[b]thiophene-3-carboxylic acid, 5-fluorobenzo[b]thiophene-3-carboxylic acid, 6-fluorobenzo[b]thiophene-3-carboxylic acid, 4-hydroxybenzo[b]thiophene-3-carboxylic acid, 5-hydroxybenzo[b]thiophene-3-carboxylic acid, 6-hydroxybenzo[b]thiophene-3-carboxylic acid, 7-hydroxybenzo[b]thiophene-3-carboxylic acid, 5-acetoxybenzo[b]thiophene-3-carboxylic acid, benzo[b]thiophene-3-carboxylic acid, 5-benzosulfonyloxybenzo[b]thiophene-3-carboxylic acid, 5-mehtylbenzo[b]thiophene-3-carboxylic acid, 6-methylbenzo[b]thiophene-3-carboxylic acid, 5-methoxybenzo[b]thiophene-3-carboxylic acid and 6-methoxybenzo[b]thiophene-3-carboxylic acid.

Each of those carboxylic acids may have the above-defined substituent.

Those carboxylic acids can be produced in accordance with the methods described in *Nippon Kagaku Zasshi*, volume 88, no. 7, pages 758-763 (1967), *Nippon Kagaku Zasshi,* volume 86, no. 10, pages 1067-1072 (1965), *J. Chem. Soc. (c),* pages 1899-1905 (1967), *J. Heterocyclic Chem.,* volume 10, pages 679-681 (1973), *J. Heterocyclic Chem.,* volume 19, pages 1131-1136 (1982) and *J. Med. Chem.,* volume 29, pages 1637-1643 (1986).

The reactive derivative of the carboxylic acid represented by the formula (III) means the corresponding acid halide (such as chloride, bromide and iodide), acid anhydride (such as that of formic acid or of mixed acid with acetic acid), activated ester (such as succinimide ester), etc. and includes an acylating agent which is usually used for acylation of amino group. In order to prepare an acid halide for example, acid may be reacted with thionyl halide (such as thionyl chloride), phosphorus halide (such as phosphorus trichloride and phosphorus pentachloride), oxalyl halide (such as oxalyl chloride), etc. according to a known method (such as *Shin Jikken Kagaku Koza* [New Experimental Chemistry], volume 14, page 1787 (1978); *Synthesis,* pages 852-854 (1986); and *Shin Jikken Kagaku Koza*, volume 22, page 115 (1992)).

The reaction may be carried out according to the condition for common acylation reaction for amino group. For example, in the case of condensation reaction using an acid halide, the reaction may be carried out using ether type solvent (such as diethyl ether, tetrahydrofuran and dioxane), benzene type solvent (such as benzene, toluene and xylene), halogenated hydrocarbon type solvent (such as dichloromethane, dichloroethane and chloroform) and others such as ethyl acetate, dimethylformamide, dimethyl sulfoxide and acetonitrile, etc. as a solvent The reaction may be carried out under cooling to at room temperature or heating or, preferably, from -20°C to ice cooling or room temperature to a heating/refluxing temperature of the reaction system for several minutes to several tens minutes, preferably 0.5 to 24 hour(s) or, more preferably, 1 to 12 hour(s), if necessary, in the presence of a base (such as an organic base [e.g., triethylamine, pyridine, N,N-dimethylaminopyridine and N-methylmorpholine] or an inorganic base [e.g., sodium hydroxide, potassium hydroxide and potassium carbonate]. When a carboxylic acid is not used as a reactive derivative but in a free form, the reaction is conducted in the presence of a condensing agent used for condensation reaction of amine with carboxylic acid (such as dicyclohexylcarbodiimide (DCC), 1-ethyl-3-(3-dimethylaminopropyl)carbodiimide or N,N'-carbonyldiimidazole).

The pharmaceutical composition of the present invention may use both an inhibitor for hematopoietic prostaglandin D synthase (H-PGDS) and an antagonist for prostaglandin D receptor as active ingredients.

Although the compounds used for treatment or prevention of brain injury used in the present invention are able to be selected from H-PGDS inhibitors or prostaglandin D receptor antagonists as mentioned above, it is also possible to screen as follows.

Thus,
1) traumatic injury is applied to brain of transgenic mouse in which human H-PGDS is expressed in large amounts,
2) a candidate compound is administered to the transgenic mouse before or after applying the traumatic injury and
3) the state of the traumatic injury in the mouse is compared with the state in a transgenic mouse to which no candidate compound is administered.

A method for the production of a transgenic mouse in which human H-PGDS is expressed in large amounts is disclosed in an international application PCT/JP00/06963 (WO 01/24627) filed on October 5, 2000. The whole document thereof constitutes a part of the present specification.

### Examples

### Preparation Example 1

### Preparation of a human hematopoietic prostaglandin D synthase over-expressing transgenic mouse

A human hematopoietic prostaglandin D synthase over-expressing transgenic mouse was prepared according to a method disclosed in WO 01/24627.

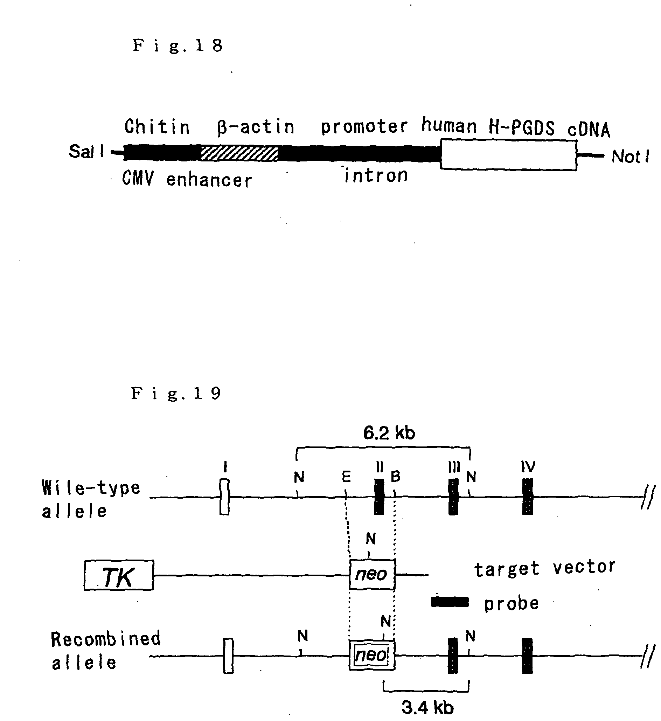

From a cDNA library prepared from mRNA of human cells, cDNA of human h-PGDS *(Eur. J. Biochem.* 267:3315-3322, 2000; GenBank Accession No. NM-014485) was cloned using cDNA of rat H-PGDS gene (*Cell* 90:1085-10975, 1997; GenBank Accession No. D 82071) as a probe. Then, cDNA of human H-PGDS was inserted into a cloning site (Sal I/Not I) of a vector pCAGGS *(Gene* 108:193-199 (1991)) to construct a transducing vector. Fig. 18 is a construction of transgene in this transducing vector. The transgene has a CMV enhancer and a chitin β-actin promoter at the upper stream of H-PGDS cDNA and, when it is transduced into chromosome of a mouse, H-PGDS mRNA is over-expressed by the action of those enhancer and promoter. The transducing vector was infused into fertilized egg of FVB mouse (obtained from the National Institute of Health Animal Genetic Resource) by a microinjection method. The fertilized egg into which gene was transduced was transplanted to oviduct of an acting parent by a common method, subjected to ontogeny and was born. DNA was extracted from tail of the resulting mouse and, using a probe which was synthesized depending upon a sequence of transgene, a transgenic mouse was selected by a Southern blot technique.

### Preparation Example 2

### Preparation of a hematopoietic prostaglandin D synthase knockout (H-PGDS KO) mouse

A hematopoietic prostaglandin D synthase knockout mouse was prepared according to a method taught in the Japanese Patent Application No. 2002/18666 filed on January 28, 2002.

A region including exon II (protein translation initiation region of H-PGDS) of known mouse H-PGDS gene was substituted with Neo^{r} gene, then a mutant sequence was prepared by integration of thymidine kinase gene of herpes virus (HSV-tk gene) into about 7 Kb upstream of H-PGDS gene and the mutant sequence was integrated into a vector to prepare a targeting vector (refer to Fig. 19).

Targeting vector was transduced at the rate of 48 µg/ml into a non-differentiated incubated ES cells (1.2 × 10⁷ cells) by electroporation to prepare ES cells into which gene was transduced. The cells were sown on a plate, G418 and ganciclovir were added to the medium after 2 days and incubation was conducted for 7 days more to prepare colonies showing a resistance to G418 and ganciclovir. Those colonies were individually separated and further incubated, DNA was extracted and a homologous recombinant ES cells were selected by a Southern blot technique.

After that, the homologous recombinant ES cell was infused into blastocyst of a mouse of C57BL/6 strain by a common method, transplanted to an acting parent and subjected to ontogeny.

As a result, 10 chimera mice were obtained. Among the resulting chimera mice, a female individual was crossed with a female wild-type mouse of C57BL/6 strain to give first generation (F₁) mice. From those F₁ mice, individuals (male/female) where mutant sequence was confirmed in one of diploid chromosomes by a Southern blot analysis were selected and crossed to give secondary generation (F₂) mice.

Finally, from those F₂ mice, individuals where mutant sequence was confirmed in both dipolar chromosomes (homozygotes) and individuals where mutant sequence was confirmed in one of dipolar chromosomes (heterozygotes) were selected by a Southern blot analysis to prepare H-PGDS knockout mice.

### Example 1

### Transduction of hematopoietic prostaglandin D synthase and DP receptor in hereditary demyelinating disease

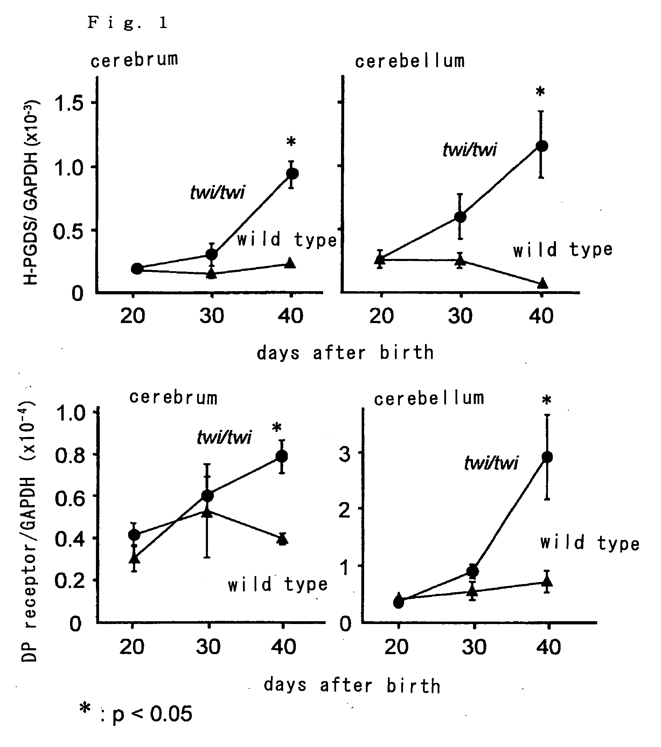

Changes in mRNA of H-PGDS and DP receptor as a result of demyelination were quantified by a quantitative RT-PCR method using a model mouse Twitcher of human Krabbe diseases which is a disease where galactosylceramidase is deficient (Kobayashi T, et al., *Brain Res.,* 202:479-483, 1980; Duchen LW, et al., *Brain,* 103:695-710, 1980; Sakai N, et al., *J. Neurochem.,* 66:1118-1124, 1996; Taniike M et al., *J. Neuropathol. Exp. Neurol*., 58:644-653, 1999) (refer to Fig. 1). Expression of mRNA of both H-PGDS and DP receptor increased as a result of demyelination.

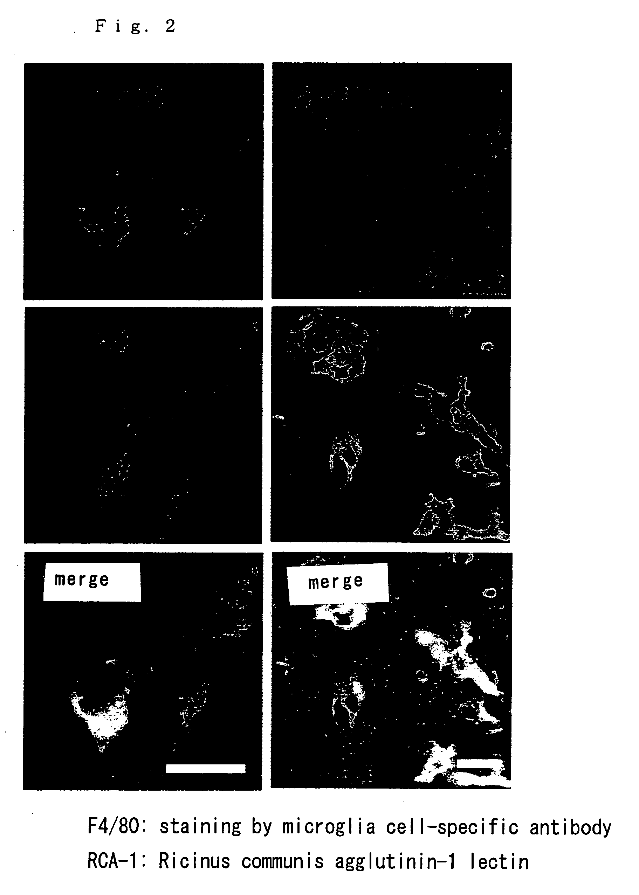

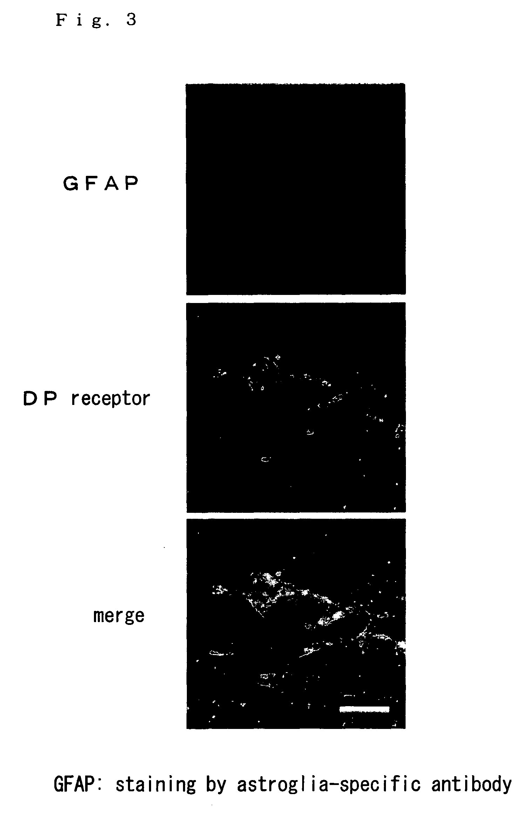

It was identified by an immunolohistochemical staining that H-PGDS was expressed in microglia cells, macrophage cells and ameboid cells accumulated in local tissues where demyelination progressed (refer to Fig. 2). On the other hand, it was identified that DP receptor was expressed in activated astroglia cells distributed around the tissues where demyelination progressed (refer to Fig. 3).

### Example 2

### Transduction of hematopoietic prostaglandin D synthase and DP receptor in autoimmune demyelinating diseases

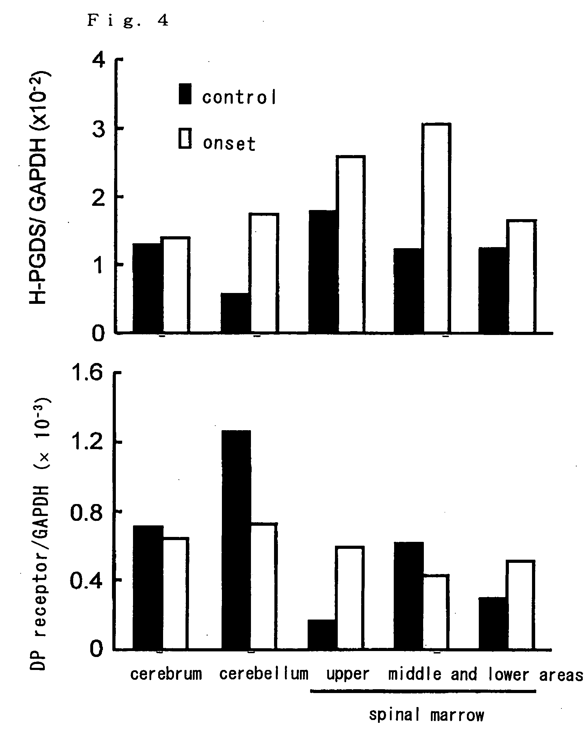

In experimental autoimmune encephalomyelitis mouse which is a model of human multiple sclerosis (Ichikawa M., et al., *Cell Immunol.,* 191:97-104, 1999; Bernhard Hemmer, et al., *Nature Review Neuroscience,* 3:291-301, 2002), expression of both H-PGDS and DP receptor as measured by a quantitative RT-PCR method also showed an increase correlated to the extent of demyelination (refer to Fig. 4).

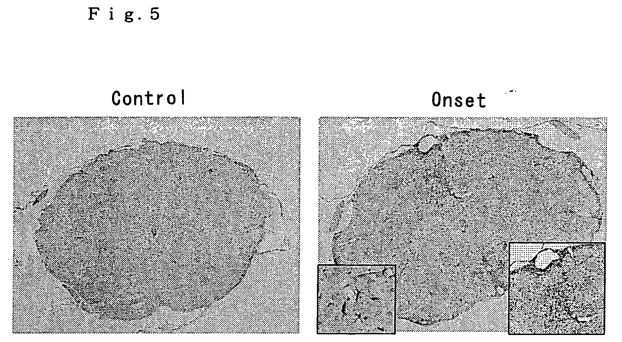

In an observation by an immunohistochemistry, H-PGDS was expressed in microglia cells, macrophage and ameboid cells accumulated in local tissues where demyelination progressed (refer to Fig. 5).

### Example 3

### Transduction of hematopoietic prostaglandin D synthase and DP receptor in traumatic brain injury

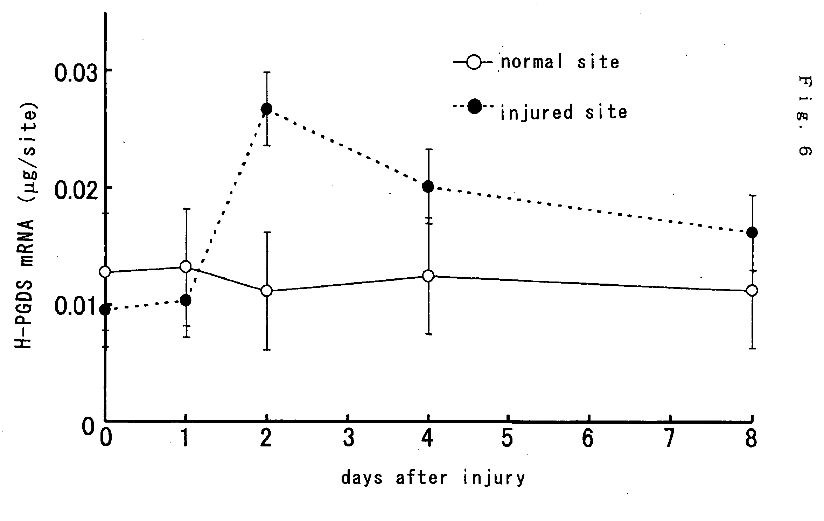

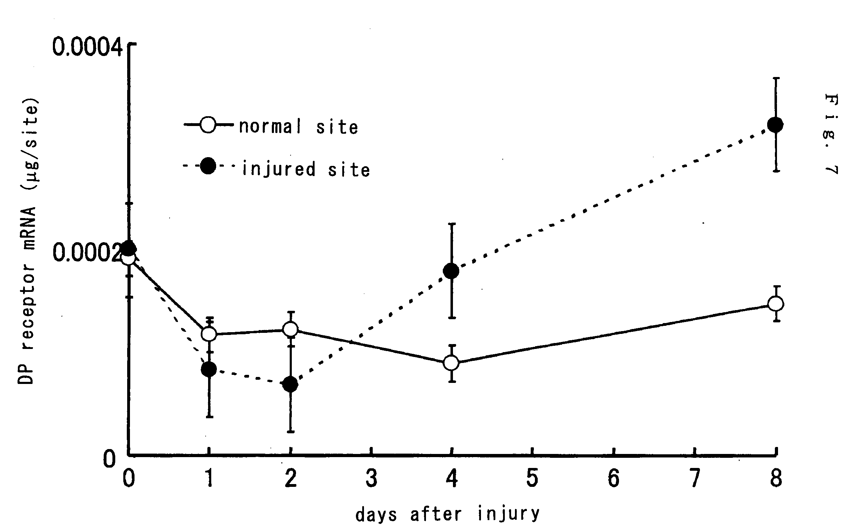

As a result of investigation of expression of mRNA of H-PGDS and DP receptor in brain injury using a model of traumatic injury of cerebral cortex (Stab would) (Salhia B, et al., *Brain Res.,* 888:87-97, 2000; Asahi M., et al., *J. Neurosci.,* 21:7724-7732, 2001; Garcia de Yebenes E., et al., *J. Neurochem.,* 73:812-820, 1999), H-PGDS showed its peaked at two days from injury (refer to Fig. 6) while DP receptor continuously increased from the second to the eighth days (refer to Fig. 7).

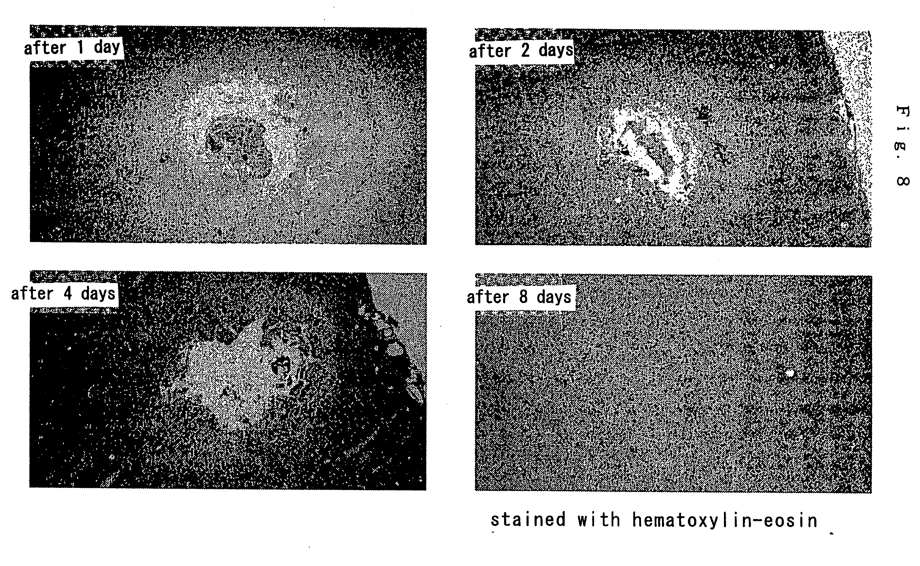

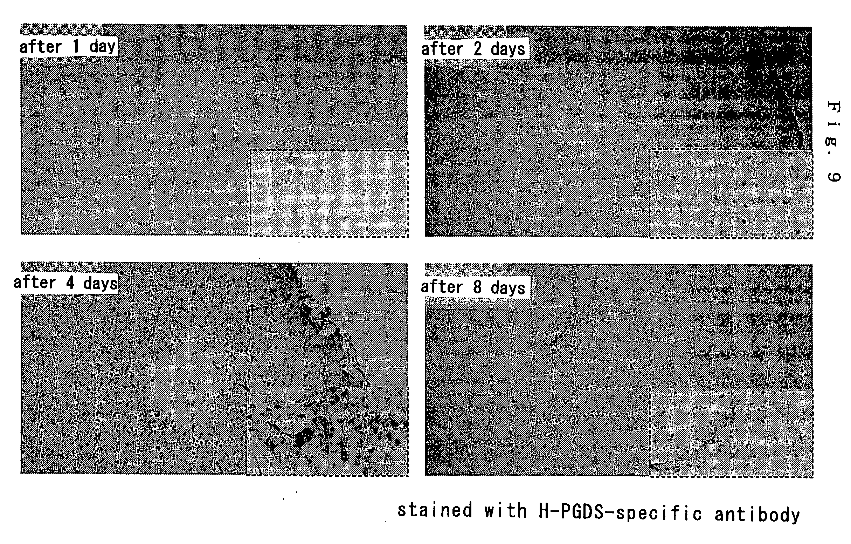

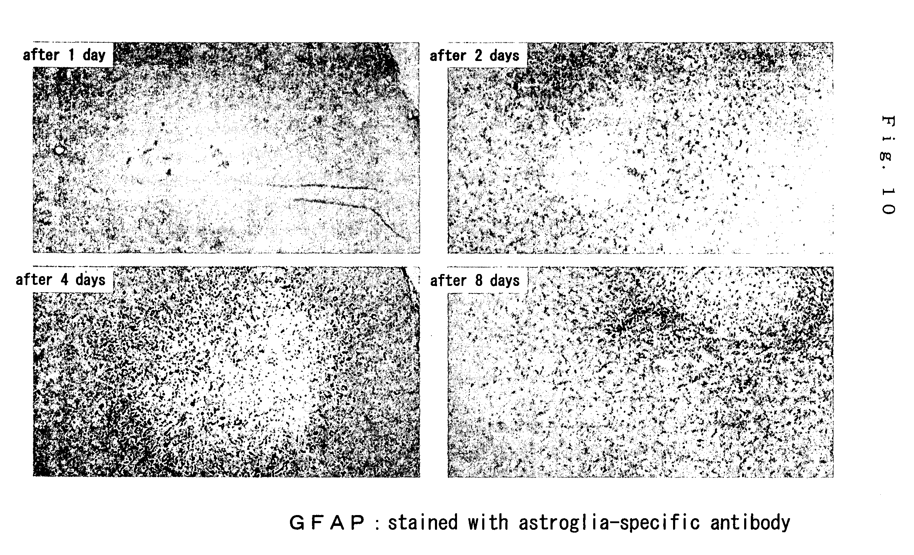

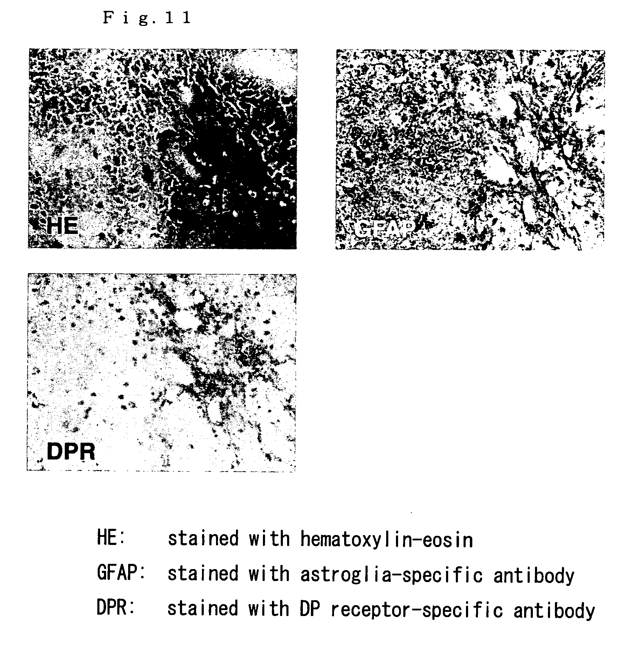

After 24 hours from the injury, transduction of H-PGDS took place in macrophage and microglia cells accumulated around the injured site (refer to Fig. 8 and Fig. 9) while, in astroglia cells around the injured site, expression of GFAP and DP receptor was enhanced and those phenomena continued until 8 days after being injured (refer to Fig. 10 and Fig. 11).

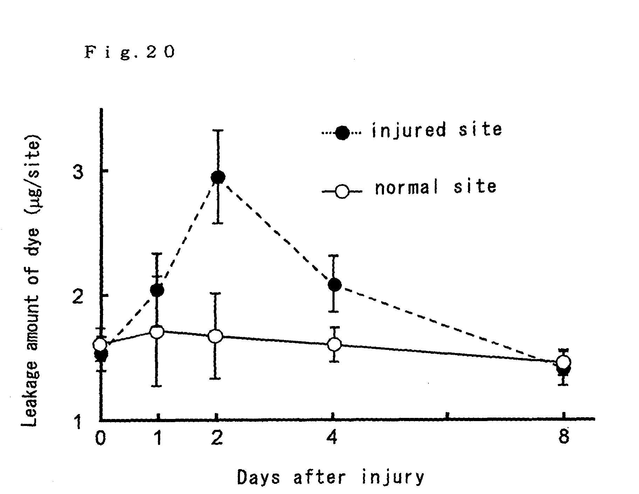

Degree of injury was quantified by a dye leakage (Evans Blue) to the injured site (Kakimura Y, et al., *Nature Medicine,* 4:1078-1080, 1998). After2 days frominjury, themaximumvalue was noted and, after that, it lowered as a result of recovery (refer to Fig. 20).

### Example 4

### Suppression of activation of astroglia cells in hereditary demyelinating disease by administration of inhibitor for hematopoietic prostaglandin D synthase

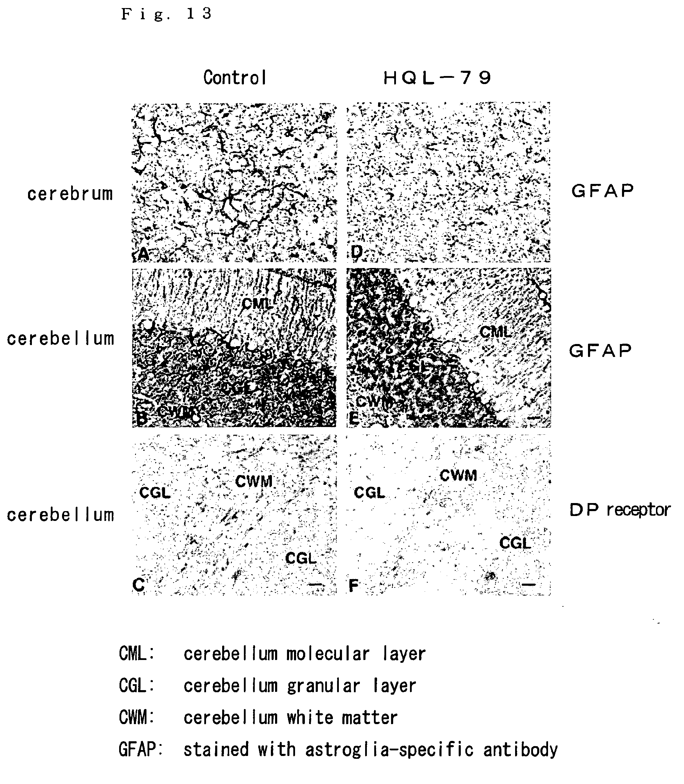

HQL-79 (4-benzhydryloxy-1-{3-(1H-tetrazol-5-yl)-propyl}piperidine) which is an H-PGDS inhibitor was subcutaneously administered to Twitcher mice at the dose of 30 mg/kg/day for 14 days whereupon activation of astroglia cells was suppressed and, at the same time, expression of DP receptor in astroglia cells lowered (refer to Fig. 13).

### Example 5

### Promotion of recovery of brain injury and suppression of transduction of DP receptor in traumatic brain injury by administration of inhibitor for hematopoietic prostaglandin D synthase

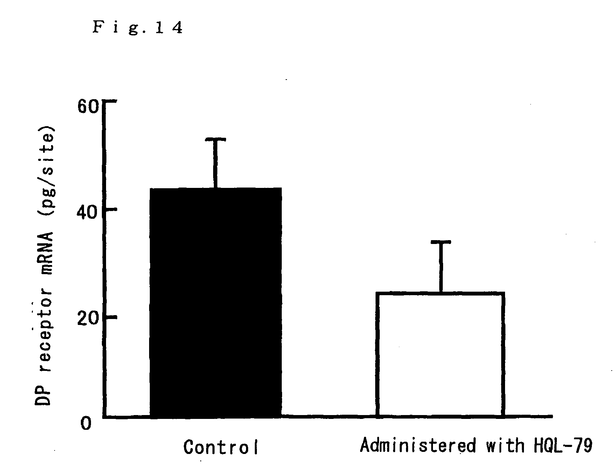

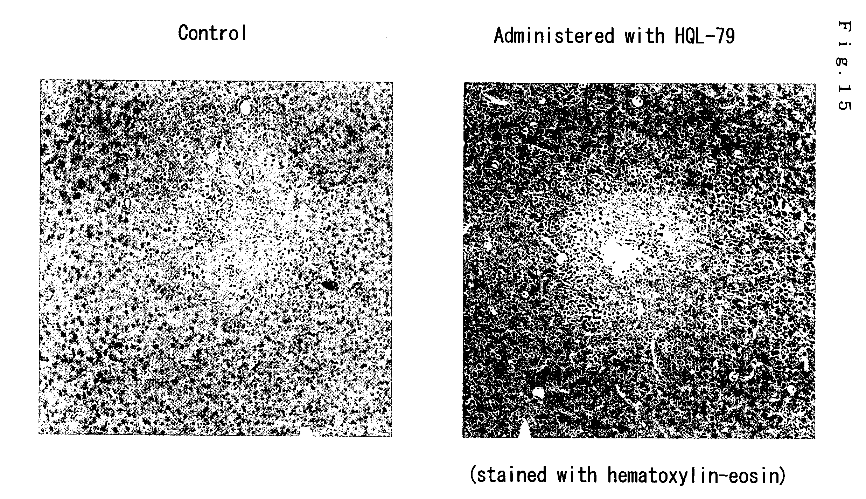

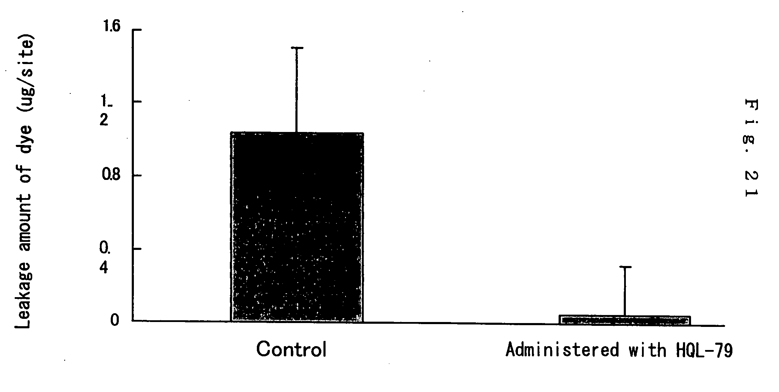

HQL-79 which is an H-PGDS inhibitor was orally administered to mice at the dose of 30 mg/kg/day for 4 days from one hour before injuring whereupon amount of mRNA of DP receptor in tissue injury region in a Stab wound model lowered (refer to Fig. 14) and promotion of recovery of brain injury was noted (refer to Fig. 15). Such a therapeutic effect was also confirmed by an experiment using a leakage reaction of dye to the injured site as an index (refer to Fig. 21).

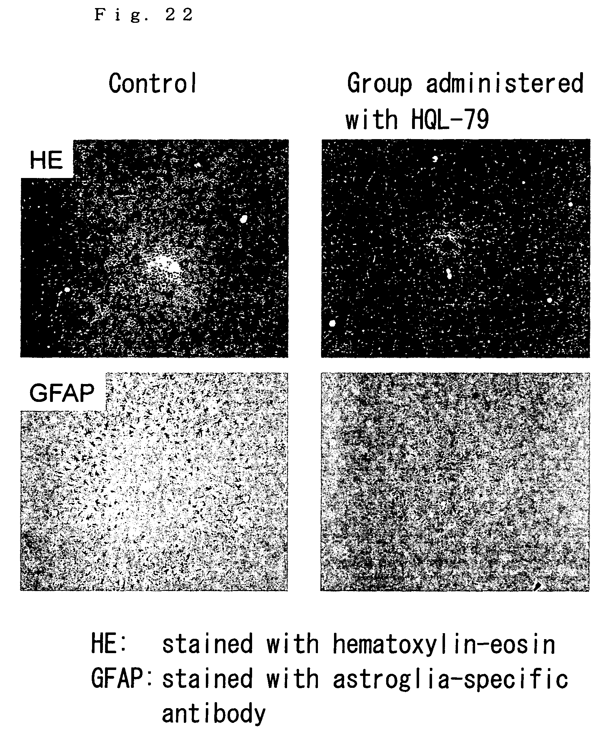

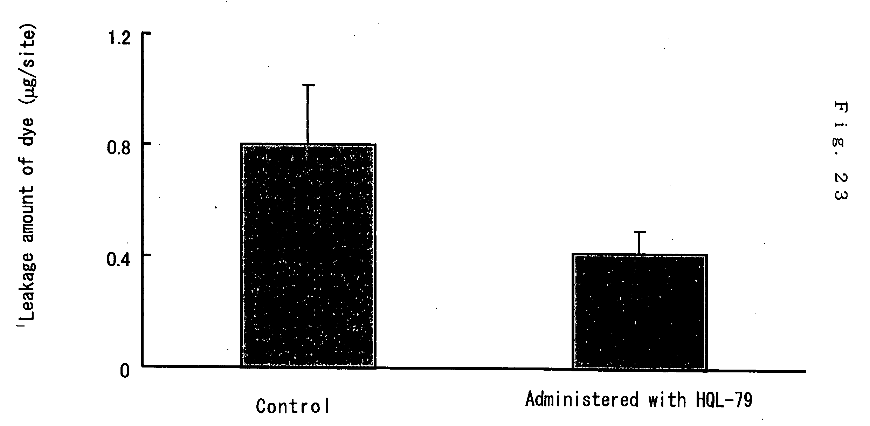

It was further confirmed that, even when administration of an H-PGDS inhibitor was initiated after injury was applied, expansion of injury was suppressed (refer to Fig. 22 and Fig. 23).

### Example 6

### Reduction in traumatic brain injury by administration of antagonist for prostaglandin D receptor

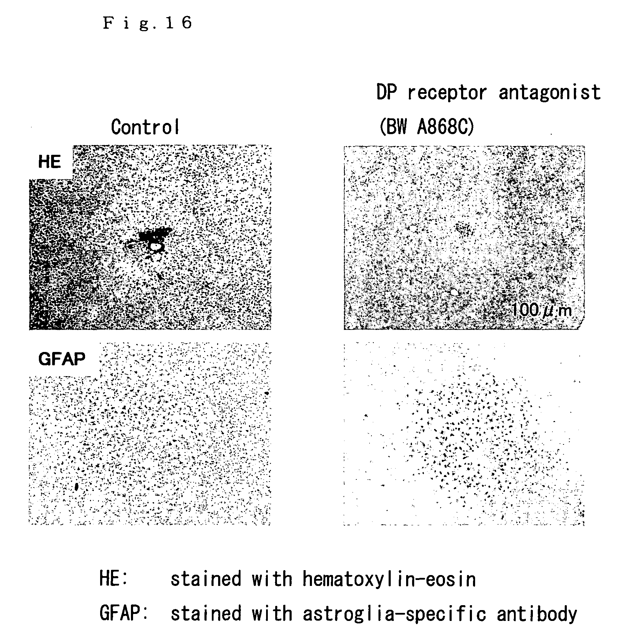

When BW A868C ((±)-3-benzyl-5-(6-carboxyhexyl)-1-(2-cyclohexyl-2-hydroxyethylamino)hydantoin) which is a DP receptor antagonist was intravenously administered to Stab wound model mice at the dose of 1 mg/kg/day for 4 days from the initial day of injury, promotion of recovery of brain injury was noted and activation of astroglia around the tissue injury site (refer to Fig. 16).

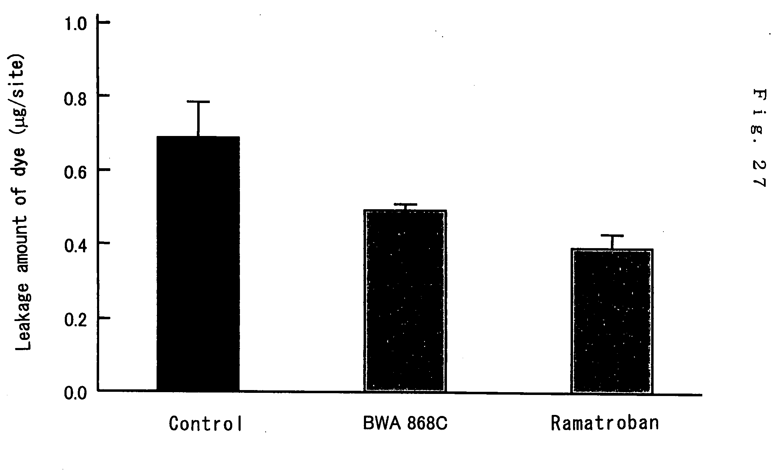

An effect of BW A868C or ramatroban ((±)-(3R)-3-(4-fluorobenzenesulfonamide)-1,2,3,4-tetrahydrocarbazol-9-propionic acid) which is an antagonist for prostaglandin D receptor to Stab wound model mice was evaluated using leakage amount of dye to the injured site as an index. Thus, Evans Blue dye was intravenously administered after 2 days from occurrence of brain injury and the amount of leakage of the dye to the tissue during 2 hours thereafter was measured. When BW A868C (1 mg/kg) was intravenously administered after 3 hours and after one day from occurrence of the brain injury, leakage of the dye to the injured site was suppressed (refer to Fig. 27).

When ramatroban was orally administered (30 mg/kg) after 3 hours and after one day from the occurrence, leakage of the dye to the injured site was suppressed (refer to Fig. 27).

### Example 7

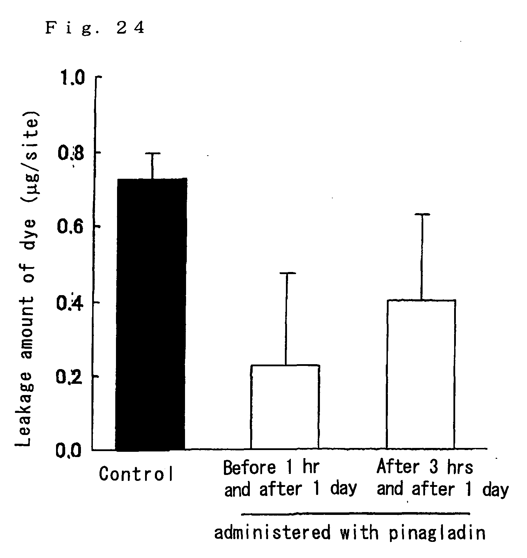

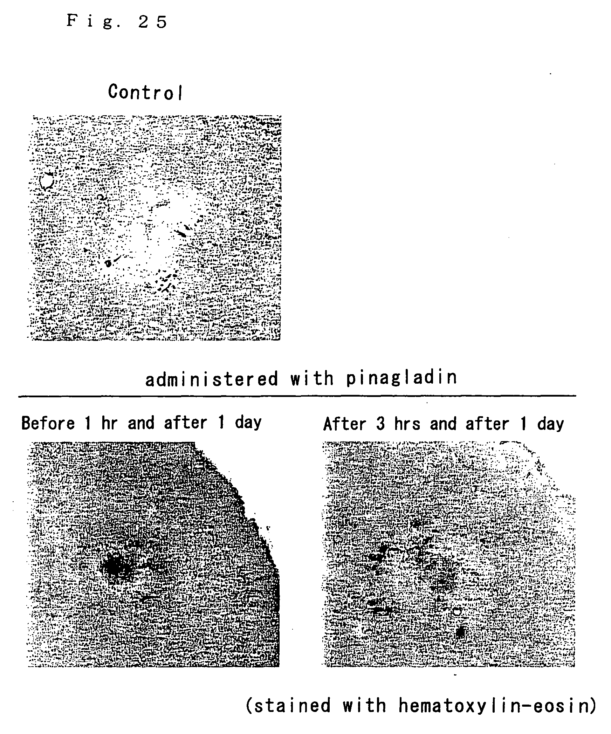

An effect of pinagladin which is a DP receptor antagonist to a Stab wound model was evaluated by a leakage amount of dye to the injured site. Thus, Evans Blue was intravenously injected on the second day after occurrence of brain injury and leakage amount of the dye into the tissue during 2 hours thereafter was measured. As a result, when pinagladin (10 mg/kg) was orally administered before 1 hour and after one day of the occurrence of the injury, an increase in leakage amount of Evans Blue dye noted as a result of brain injury was suppressed (refer to Fig. 24). In a further histopathological investigation, pinagladin suppressed the progress of brain injury in a Stab wound model in any of administration routes (refer to Fig. 25).

### Example 8

### Exacerbation of traumatic brain injury by human hematopoietic prostaglandin D synthase over-expression

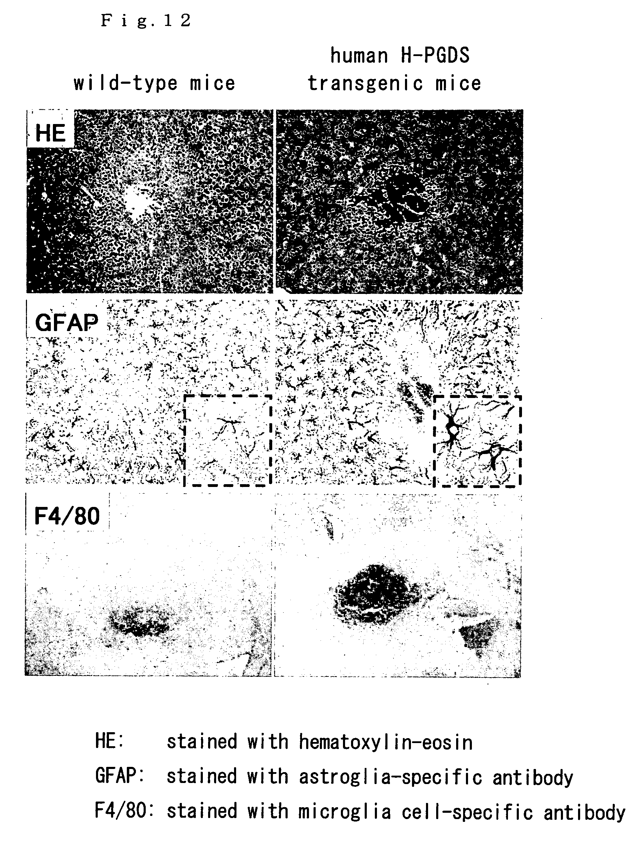

In a Stab wound model using transgenic mice where human H-PGDS was expressed in large amounts prepared in Preparation Example 1, accumulation of macrophage in injured site and activation of astroglia cells which was immunohistochemically tested were significant as compared with those in mild-type mice whereby recovery delayed (refer to Fig. 12).

### Example 9

### Reduction in traumatic brain injury by hematopoietic prostaglandin D synthase gene deficiency

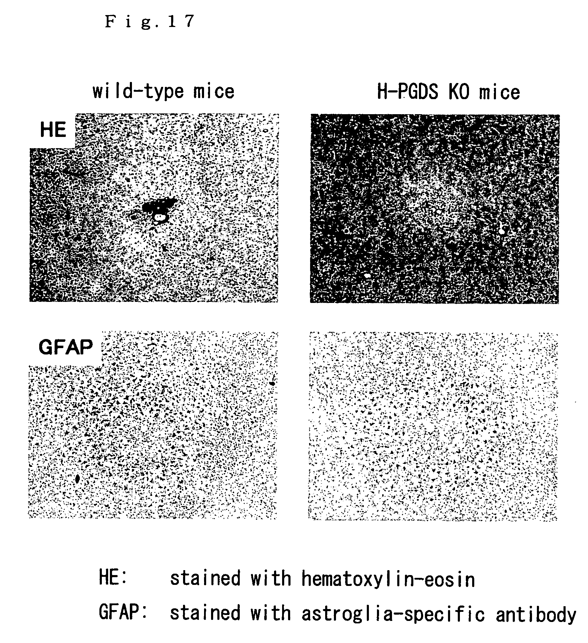

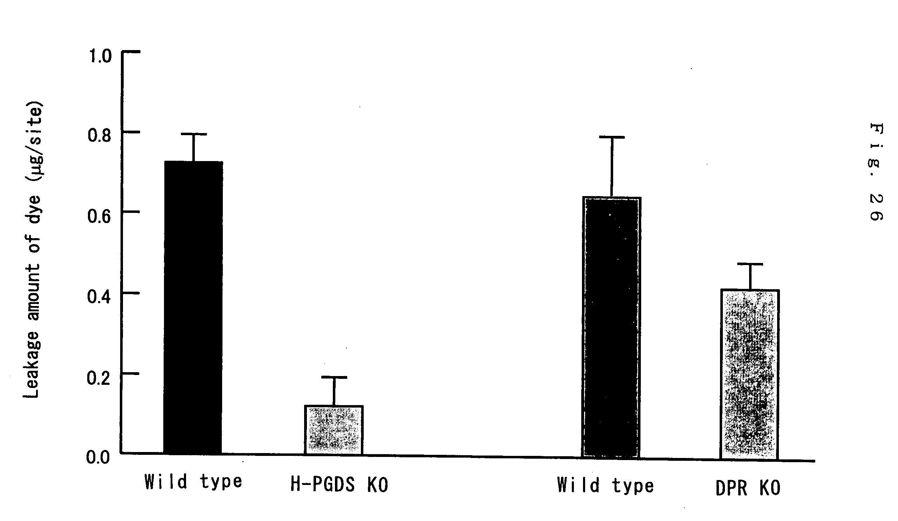

In a Stab wound model using hematopoietic prostaglandin D synthase knockout (H-PGDS KO) mice (homozygote) prepared in Preparation Example 2, bleeding in the injured site, activation of astroglia histoimmunochemically checked using an anti-GFAP antibody and dye leakage at the injured site were slight as compared with those in mice of a wild type (refer to Fig. 17 and Fig. 26).

On the other hand, in a Stab wound model using DP receptor knockout (DPR KO) mice (Matsuoka T, et al., *Science,* 17;287(5460):2013-2017, 2000), leakage of the dye at the injured site was slight as compared with that in mice of a wild type.

When the inhibitor for hematopoietic prostaglandin D synthase (H-PGDS) and/or the antagonist for prostaglandin D receptor according to the present invention are/is used for the treatment, they/it are/is made into pharmaceutical composition for oral or parenteral administration. A pharmaceutical composition comprising the inhibitor for hematopoietic prostaglandin D synthase (H-PGDS) and/or the antagonist for prostaglandin D receptor according to the present invention may be in oral and parenteral dosage forms.

Thus, they may be made into formulation for oral administration such as tablets, capsules, granules, powder and syrup or into formulation for parenteral administration such as injection solution or suspension for intravenous injection, intramuscular injection, subcutaneous injection, etc., inhalant, eye drop, nose drop, suppository, preparation for percutaneous administration and percutaneous absorption such as ointment, poultice and cataplasm. Preferably, agents for oral administration or drugs for injection are used.

Those formulation can be manufactured using appropriate carriers, excipients, solvent, substrates, etc. which have been known by persons skilled in the art. In the case of tablets, for example, active ingredient and auxiliary components are compressed or molded together. As the auxiliary components, there may be used pharmaceutically acceptable excipients such as binder (e.g., corn starch), filler (e.g., lactose and microcrystalline cellulose), disintegrating agent (e.g., sodium starch glycolate) and lubricant (e.g., magnesium stearate). Tablets may be appropriately coated. In the case of liquid preparations such as syrup, liquid and suspension, there may be used suspending agent (e.g., methyl cellulose), emulsifier (e.g., lecithin), preservative, etc. In the case of the formulation for injection, any of the forms of solution, suspension and oily or aqueous emulsion may be used and they may contain, for example, a dispersing agent or a stabilizer for suspension. In the case of the formulation for percutaneous administration and percutaneous absorption such as ointment, poultice and cataplasm, the formulation is prepared using an aqueous substrate (water, lower alcohol, polyol) or an oily substrate (higher fatty acid esters (isopropyl myristate), lipophilic alcohol).

Although the dose of the hematopoietic prostaglandin D synthase (H-PGDS) inhibitor and/or the prostaglandin D receptor antagonist to be administered according to the present invention may vary depending upon dosage form, symptom, age, body weight or sex of a patient or drug(s) used together (if any) and are/is finally subjected to the decision of medical doctors, it is administered, in the case of oral administration, in a dose of 0.01 to 100 mg, preferably 0.01 to 50 mg or, more preferably, 0.01 to 30 mg per kg of body weight while, in the case of parenteral administration, in a dose of 0.001 to 100 mg, preferably 0.001 to 5 mg or, more preferably, 0.001 to 3 mg per kg of body weight. That may be administered by dividing into one to four time(s).

### Formulation Examples

### Formulation 1

A tablet containing the following components was prepared.

| | |
|---|---|
| Compound represented by the formula (I) | 10 mg |
| Lactose | 90 mg |
| Microcrystalline cellulose | 30 mg |
| CMC-Na | 15 mg |
| Magnesium stearate | 5 mg |
| | 150 mg |

The compound represented by the formula (I), lactose, microcrystalline cellulose and CMC-Na (carboxymethyl cellulose sodium salt) were passed through a sieve of 60 meshes and mixed. Magnesium stearate was mixed with the above mixture to give mixed powder for the manufacture of tablets. The mixed powder was directly compressed to give tablets each weighing 150 mg.

### Formulation 2

A suspension containing 50 mg of the active ingredient was prepared as follows:

| | |
|---|---|
| Compound represented by the formula (I) | 50 mg |
| Carboxymethyl cellulose sodium | 50 mg |
| Syrup | 1.25 ml |
| Benzoic acid solution | 0.10 ml |
| Perfume | q.v. |
| Dye | q.v. |
| Pure water was added to make | 5 ml |

The active ingredient was passed through a sieve of No. 45 mesh U. S. and mixed with carboxymethyl cellulose sodium and syrup to give a smooth paste. Then benzoic acid solution and perfume were diluted with a part of water and added thereto followed by stirring. After that, sufficient amount of water was added to give a necessary volume.

### Formulation 3

Formulation for intravenous administration was manufactured as follows.

| | |
|---|---|
| Compound represented by the formula (I) | 100 mg |
| Saturated fatty acid glyceride | 1000 ml |

Usually, a solution comprising the above components is intravenously administered to a patient at the rate of 1 ml per minute.

### Formulation 4

A gelatin hard capsule preparation of the following composition was prepared by a conventional method.

| | |
|---|---|
| HQL-79 | 10 mg |
| Starch | 50 mg |
| Magnesium stearate | 10 mg |

### Formulation 5

The following tablet was prepared by a conventional method.

| | |
|---|---|
| HQL-79 | 10 mg |
| Cellulose, microcrystalline | 500 mg |
| Silicon dioxide | 10 mg |
| Magnesium stearate | 10 mg |

As fully illustrated hereinabove, the composition of the present invention is able to be used for the treatment of diseases such as cerebrovascular disorder, brain degenerative disease and demyelinating disease.

When H-PGDS transduced by microglia cells or macrophage at the part affected by brain injury is inhibited or when activity of DP receptor expressed in astroglia cells around the injured area is inhibited, it is now possible to treat or prevent the brain injury in which prostaglandin D₂ is participated.

In patients suffering from multiple sclerosis, biosynthesis of prostaglandin D₂ is active *(Science* 294:1731-11735, 2001) and there is a high possibility that it acts as an exacerbating factor during the onset step of experimental autoimmune encephalomyelitis in mice which is a model thereof. Therefore, when a specific inhibitor for H-PGDS or an antagonist for receptor is used, it is able to be a useful treating method for treatment or prevention of multiple sclerosis as a substitute for steroid therapy and immunosuppressants which have been conducted/used at present. In addition, such a drug suppresses a local inflammation reaction as a result of autoimmune cell disorder and nerve cell death whereby it is also able to be applied for the treatment of intractable diseases accompanied by neurofibrillary degeneration including Alzheimer's disease.

## Claims

1. A pharmaceutical composition used for treatment or prevention of brain injury comprising an inhbitor for hematopoietic prostaglandin D synthase (H-PGDS) as an active ingredient.

2. The pharmaceutical composition according to claim 1, wherein the H-PGDS inhibitor is 4-benzhydryloxy-1-{3-(1H-tetrazol-5-yl)-propyl}piperidine, 1-amino-4-{4-[4-chloro-6-(2-sulfo-phenylamino)-[1,3,5]-triazine-2-y lmethyl]-3-sulfo-phenylamino}-9,10-dioxo-9,10-dihydro-anthracene-2-sulfonic acid, 1-amino-4-(4-sulfamoylanilino)-anthraquinone-2-sulfonic acid or pharmaceutically acceptable salt thereof or hydrate thereof or 2-(2'-benzothiazolyl)-5-styryl-3-(4'-phthalhydrazidyl)-tetrazolium chloride or a hydrate thereof.

3. A pharmaceutical composition used for treatment or prevention of brain injury comprising an antagonist for prostaglandin D receptor as an effective ingredient.

4. The pharmaceutical composition according to claim 3, wherein the antagonist for a prostaglandin D receptor is (±)-3-benzyl-5-(6-carboxyhexyl)-1-(2-cyclohexyl-2-hydroxyethylamino)-hydantoin, (+)-(3R)-3-(4-fluorobenzenesulfonamide)-1,2,3,4-tetrahydrocarbazol-9-propionic acid, (Z)-7-[(1R,2R,3S,5S)-2-(5-hydroxybenzo[b]thiophene-3-ylcarbonylamino)-10-norpinan-3-yl]hepta-5-enoic acid, (Z)-7-[(1R,2R,3S,5S)-2-(benzo[b]-thiophene-3-ylcarbonylamino)-10-norpinan-3-yl]hepta-5-enoic acid and pharmaceutically acceptable salt thereof and hydrate thereof.

5. The pharmaceutical composition according to claim 3, wherein the antagonist for a prostaglandin D receptor is a compound represented by the formula (I) 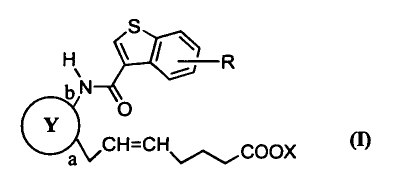 (wherein, 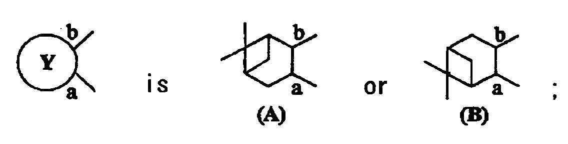 R is hydrogen, alkyl, alkoxy, halogen, hydroxyl, acyloxy or optionally substituted arylsulfonyloxy; X is hydrogen or alkyl; and a double bond of an α-chain is in an E-configuration or a Z-configuration) or a pharmaceutically acceptable salt or a hydrate thereof.

6. The pharmaceutical composition according to claim 3, wherein the antagonist for a prostaglandin D receptor is a compound represented by the formula (IA) 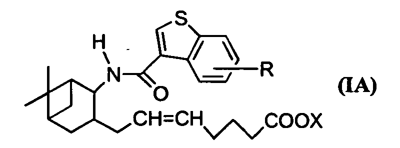 (wherein R and X have the same meanings as defined already and a double bond of an α-chain is in an E-configuration or a Z-configuration) or a pharmaceutically acceptable salt or a hydrate thereof.

7. The pharmaceutical composition according to claim 3, wherein the antagonist for a prostaglandin D receptor is a compound represented by the formula (IA-a) 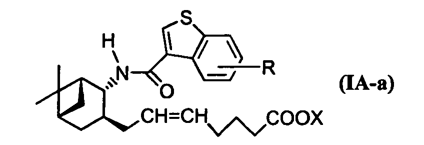 (wherein R and X have the same meanings as defined already and a double bond of an α-chain is in an E-configuration or a Z-configuration) or a pharmaceutically acceptable salt or a hydrate thereof.

8. A method for treatment of brain injury comprising administration of a hematopoietic prostaglandin D synthase (H-PGDS) inhibitor of an effective dose.

9. A use of a hematopoietic prostaglandin D synthase (H-PGDS) inhibitor for the manufacture of a drug for treatment of brain injury.

10. A method for treatment of brain injury comprising administration of a prostaglandin D receptor antagonist of an effective dose.

11. A use of a prostaglandin D receptor antagonist for the manufacture of a drug for treatment of brain injury.

12. A pharmaceutical composition to be used for treatment or prevention of brain injury comprising a hematopoietic prostaglandin D synthase (H-PGDS) inhibitor and a prostaglandin D receptor antagonist as effective ingredients.

13. A method of screening of a compound used for treatment or prevention of brain injury comprising that
1) trauma is applied to brain of a transgenic mouse where human H-PGDS is over-expressed,
2) a candidate compound is administered to the transgenic mouse before or after applying the trauma and
3) a state of the trauma in the mouse is compared with a state of a transgenic mouse to which no candidate compound is administered.
